# EUROPEAN PATENT SPECIFICATION

(11) **EP 0 468 037 B1**
(45) Date of publication and mention of the grant of the patent: **22.03.1995**
(21) Application number: 91904713.4
(22) Date of filing: 13.02.1991
(51) Int. Cl.: C07C 401/00

(54) **PROCESS FOR PREPARING VITAMIN D2 COMPOUNDS AND THE CORRESPONDING 1 ALPHA-HYDROXYLATED DERIVATIVES**
VERFAHREN ZUR HERSTELLUNG VON VITAMIN-D2-VERBINDUNGEN UND IHRER 1-ALPHA HYDROGENIERTEN DERIVATE
METHODE DE PREPARATION DE COMPOSES DE VITAMINE D2 ET LES DERIVES 1 ALPHA-HYDROXYLES CORRESPONDANTS

(30) Priority: 14.02.1990 US 481985; 14.02.1990 US 481988; 14.02.1990 US 481990
(43) Date of publication of application: 29.01.1992
(73) Proprietor: WISCONSIN ALUMNI RESEARCH FOUNDATION, Madison, WI 53705 (US)
(72) Inventor: DELUCA, Hector, Floyd, Deerfield, WI 53531 (US); SCHNOES, Heinrich, Konstantine, Madison, WI 53705 (US); OKADA, Shigeya, Hirakatashi 573 (JP)
(74) Representative: Ellis-Jones, Patrick George Armine
(86) International application number: US9100977
(87) International publication number: WO9112240

(56) References cited:
- EP-A- 0 278 732
- EP-A- 0 337 305
- WO-A-84/01155
- WO-A-84/04527
- WO-A-85/02189
- WO-A-85/03939
- BIOORGANIC CHEMISTRY, vol. 15, no. 2, June 1987, American Press, Inc.; R.R. SICINSKI et al.: "Delta 22-unsaturated analogs of vitamin D3 and their C(1)-hydroxylated derivatives", pages 152-166, see the whole document.
- TETRAHEDRON LETTERS, vol. 22, no. 27, 1981, Pergamon Press Ltd., GB; Y. YAMADA et al.: "Stereoselective synthesis of (25S)-25-hydroxyvitamin D3 26,23-lactone", p. 2591-2594, see the whole document.
- THE JOURNAL OF ORGANIC CHEMISTRY, vol. 49, no. 12, 15 June 1984, American Chemical Society; J.W. MORZYCKI et al.: "Synthesis of 25-hydroxyvitamin D2 and its 24-epimer", see p 2148-2151, see the whole document.
- PROCEDURE OF THE WORK SHOP ON VITAMIN D, 5th, 1982, Walter de Gruyter & Co.; S. YAMADA et al.: "Stereoselctive synthesis of (23R,25S) - and (23S,25R)-25-Hydroxyvitamin D3 26,23-lactone: Determination of the configuration of a metabolite of vitamin D3, calcidiol lactone", p. 1085-1087, see the whole document.

## Description

### Technical Field

This invention relates to biologically active vitamin D compounds.

More specifically, this invention relates to a process for the preparation of hydroxylated derivatives of vitamin D₂.

### Background of the Invention

The D vitamins are very important agents for the control of calcium and phosphate metabolism in animals and humans, and have long been used as dietary supplements and in clinical practice to assure proper bone growth and development. It is now known that the in vivo activity of these vitamins, specifically of vitamin D₂ and D₃, is dependent on metabolism to hydroxylated forms. Thus, vitamin D₃ undergoes two successive hydroxylation reactions in vivo, leading first to 25-hydroxyvitamin D₃ and then to 1,25-dihydroxyvitamin D₃ and the latter is thought to be the compound responsible for the well-known beneficial effects of vitamin D₃. Likewise, vitamin D₂, which is commonly used as a dietary supplement, undergoes an analogous hydroxylation sequence to its active forms, being first converted to 25-hydroxyvitamin D₂ (25-OH-D₂) and then to 1,25-dihydroxyvitamin D₂ (1,25-(OH)₂D₂). These facts are well established and well known in the art [see, for example, Suda et al. Biochemistry 8, 3515 (1969) and Jones et al. Biochemistry 14, 1250 (1975)].

Like the metabolites of the vitamin D₃ series, the hydroxylated forms of vitamin D₂ named above are, because of their potency and other beneficial properties, highly desirable dietary supplements, or pharmaceutical agents, for the cure or prevention of bone or related diseases, and their value and possible use is recognized in patents relating to these compounds (U. S. Letters Pat. Nos. 3,585,221 and 3,880,894].

Whereas many metabolites of vitamin D₃ have been prepared by chemical synthesis, there has been less work on the preparation of vitamin D₂ metabolites. The known synthetic processes for the metabolites of the D₃-series (especially as far as they relate to the preparation of side chain hydroxylated compounds) are, of course, in general not suitable for the preparation of the corresponding vitamin D₂ metabolites, since the latter are characterized by a side chain structure (i.e. presence of a double bond and an extra methyl group) which requires a different synthetic approach from that applicable to side chain hydroxylated D₃ compounds.

Various approaches for the preparation of vitamin D₂ metabolites are known, and are described in U. S. Patent Nos. 4,448,721, 4,847,012 and 4,769,181. Other preparations of 25-OH-D₂ and 1α,25-(OH)₂D₂ compounds involving condensation of side chains with a steroid nucleus are shown in Yamada et al, "Facile And Stereoselective Synthesis of 25-hydroxyvitamin D₂", Tetrahedron Letters, Vol. 25, No. 33, pp. 3347-3350, 1984 and in Tsuji et al, "A New And Convenient Synthesis of 1α,25-Dihydroxyvitamin D₂ And Its 24R-Epimer", Bull, Chem. Soc. Jpn., Vol. 62, No. 10, pp. 3132-3137, 1989. Perlman et al have reported the preparation of the epimer of 1α-OH-D₂ by condensation of a suitable side chain fragment with a vitamin D nucleus in J. Chem. Soc. Chem. Com. pp. 1113-1115, 1989.

### Disclosure of Invention

A novel and convenient synthesis of vitamin D₂ compounds has now been developed and this forms the subject of the present invention. This synthesis provides vitamin D₂ compounds characterized by the structures shown below where X₂ is hydrogen, such as vitamin D₂ itself and the 24-epimer of vitamin D₂, namely 24-epi-vitamin D₂ (24-epi D₂), characterized by the structures shown below where X₁ and X₂ are both hydrogen and R₁, R₂ and R₃ are methyl. This synthesis also provides 25 hydroxylated vitamin D₂ compounds, such as 25-hydroxyvitamin D₂ (25-OH-D₂) and the 24-epimer of 25-hydroxyvitamin D₂, namely 25-hydroxy-24-epi-vitamin D₂ (25-OH-24-epi D₂), characterized by the structures shown below where X₁ is hydrogen, X₂ is hydroxy and R₁, R₂ and R₃ are methyl. 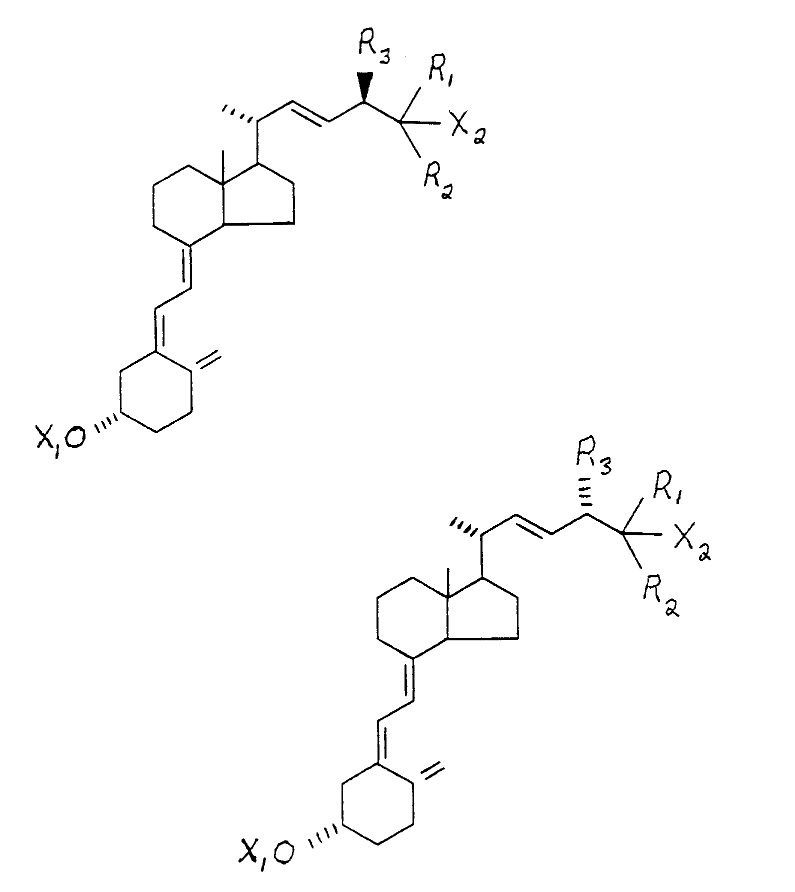
This synthesis thus provides compounds where X₂ may be either hydrogen or hydroxy, as well as the corresponding alkyl or aryl analogs thereof, characterized by the structures above where R₁ and R₂ are alkyl or aryl, and the corresponding side chain substituted derivatives where R₃ is alkyl, hydroxy, protected hydroxy (as defined below for X₁ and X₂), hydrogen or fluorine, and the hydroxy-protected derivatives of these compounds characterized by the structures above, where X₁ is selected from the group consisting of acyl, alkylsilyl, or alkoxyalkyl and X₂ is selected from the group consisting of O-acyl, O-alkylsilyl, or O-alkoxyalkyl.

In addition, the present process provides the 5,6-trans-isomers of the above compounds. Furthermore, the above compounds can be 1α-hydroxylated by known methods, so as to produce the corresponding 1α-hydroxyvitamin D derivatives. Especially preferred examples of the latter are 1α-hydroxyvitamin D₂, 1α,25-dihydroxyvitamin D₂, 1α-24-epi-vitamin D₂ and 1α,25-dihydroxy-24-epi-vitamin D₂.

The term "acyl", as used in this specification, signifies an aliphatic acyl group of from 1 to about 6 carbons, in all possible isomeric forms (e.g. formyl, acetyl, propionyl, butyryl, isobutyryl, valeryl, etc.), or an aromatic acyl group (aroyl group) such as benzoyl, the isomeric methyl-benzoyls, the isomeric nitro- or halo-benzoyls, or a dicarboxylic acyl group of from 2 to 6 atoms chain length, i.e. acyl groups of the type ROOC(CH₂)ₙCO-, or ROCCH₂-O-CH₂CO-, where n has values between 0 and 4 inclusive and R is hydrogen or an alkyl radical, such as oxalyl, malonyl, succinoyl, glutaryl, adipyl, diglycolyl. The term "alkyl" refers to an alkyl group of 1 to 6 carbons in all posible isomeric forms, e.g. methyl, ethyl, propyl, isopropyl, butyl, isobutyl, sec.-butyl, pentyl, etc., and the work "aryl" signifies a phenyl or substituted phenyl group, e.g. alkylphenyl, methoxyphenyl. The term "alkylsilyl" refers to trialkyl silicone groupings where the alkyl groups may be the same or different as exemplified by trimethylsilyl, triethylsilyl, dimethyl-tert.-butylsilyl and similar groupings. The term "alkoxyalkyl" refers to protecting groups such as methoxymethyl, ethoxymethyl, methoxyethoxymethyl, and similar alkoxymethyl groups, as well as the related cyclic structures, such as tetrahydropyranyl or tetrahydrofuranyl.

The overall process developed for the preparation of the above compounds may be divided into two general phases, namely (a) addition of a completed, preformed side chain fragment to a suitable steroidal precursor to produce a 5,7-diene steroid as the central intermediate, and (b) conversion of this 5,7-diene to the vitamin D structure to produce the desired vitamin D compound, and, if desired, (c) further conversion of the latter product to the corresponding 1α-hydroxyvitamin D compound. This process avoids the relatively difficult step of isomer separation which is required after conversion in the process of U. S. patent No. 4,448,721. The process of the present invention also increases the yield of the end product since it utilizes a completed, preformed pure isomer side chain fragment to make the desired end product, rather than a mixture or side chain isomers as in U. S. Patent No. 4,448,721.

Accordingly, the process of this invention prepares a Vitamin D compound of formula: 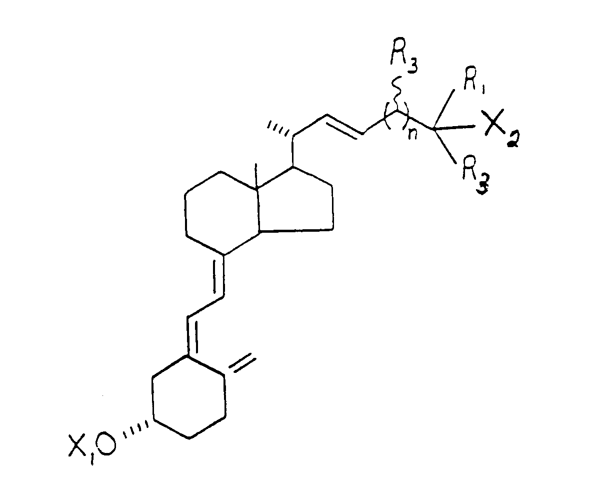
where R₃ may have the R or S configuration,
n is an integer from 1 to 5, R₃ is alkyl, hydroxy, protected hydroxy, hydrogen or flourine,
and R₁ and R₂ are each independently alkyl or aryl, with the proviso that when n=1 and R₃ is hydrogen, R₁ and R₂ cannot both be methyl,
which comprises the reaction of a steroidal 22-aldehyde of the structure: 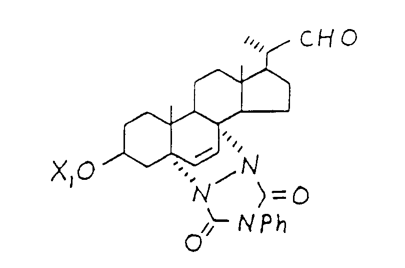
where X₁ is hydrogen or a hydroxy-protecting group, with a sulfone derivative of the general formula,

ArSO₂CH₂R

where Ar represents an aryl group, typically a phenyl or tolyl group, and R is straight or branched substituted or unsubstituted, alkyl radicals typically of from 1 to 25 carbon atoms, where the substituents are hydroxy, protected hydroxy, and fluorine, namely alkyl, hydroxylated alkyl, hydroxy-protected hydroxylated alkyl, fluoro-substituted alkyl, fluoro-substituted hydroxylated alkyl, fluoro-substituted hydroxy-protected alkyl.

The coupling reaction, generally conducted in a basic medium, between the above aldehyde and sulfone derivatives, yields a condensation product of the formula: 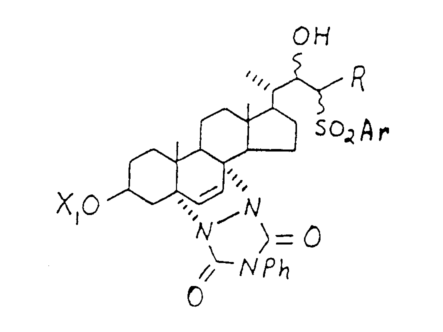
which is then subjected to reduction (either as the 22-hydroxy, or as the corresponding 22-O-acylated or sulphonated derivative), typically using metal amalgams (Na, Al, Zn amalgams or related dissolving metal reduction systems, so as to provide the 22, 23-unsaturated steroid of the formula: 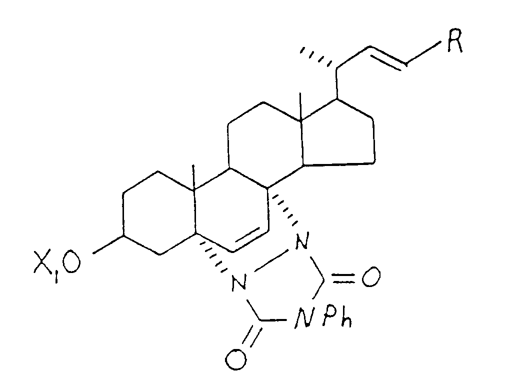
where R and X₁ represent the groupings defined above. This steroid intermediate can then be further converted by known reactions to the desired vitamin D compounds. This involves removal of the triazoline group to form a 5,7-diene steroid intermediate, subjecting this intermediate to irradiation with ultraviolet light in an ether or hydrocarbon, isomerising the resulting previtamin D compound in a solvent at a temperature from 50° to 90°C and removing, or exchanging and then removing, any hydroxy protecting group.

It is readily apparent that by suitable variation of R in the aryl sulfone derivative used in the above coupling process a range of different vitamin D compounds can be produced.

### 25-Hydroxylated Compounds (X₂ Is Hydroxy)

The reaction sequence illustrated by Process Scheme I presents a specific embodiment of the overall process, whereas Process Scheme II illustrates preparation of an appropriate side chain unit for addition to the steroid-22-aldehyde as shown in Scheme I.

Starting materials for the present process are steroidal 22-aldehydes, such as, for example, the PTAD-diene-protected-22-aldehyde (4) shown in Scheme I (where PTAD refers to the phenyltriazoline-3,5-dione-protecting group shown), which in turn can be prepared from ergosterol by the known steps (Scheme I).

The first step of this process comprises the addition of a suitable side chain fragment. Thus, condensation of aldehyde (4) with a sulfonyl-side chain fragment as shown in Scheme I (sulfone (21), further described below) present in the form of its anion, in an ether or hydrocarbon solvent, provides the hydroxysulfone intermediate (5). The anion of the sulfone (21) side chain fragment is generated by treatment of the sulfone with a strong base, such as lithium diethylamide, n-butyl lithium or methyl or ethyl magnesium bromide (or similar Grignard reagent) in an ether or hydrocarbon solvent, and to this solution of sulfone anion the steroid aldehyde (compound 4) as an ether or hydrocarbon solution is then added. The reaction is best effected under an inert atmosphere.

The next step comprises the removal of the hydroxy-and phenylsulfonyl groups in the side chain with formation of the 22(23)-trans-double bond. Thus, treatment of compound (5), in methanol solution saturated with NaHPO₄, with sodium amalgam under an inert atmosphere, gives compound (6) featuring the desired trans-22-double bond in the side chain. If desired, the 22-hydroxy group in compound (5) can also be acylated or sulfonylated (e.g. mesylated) prior to the Na/Hg-reduction step, but this is not generally required.

It is to be noted that, as shown in Process Scheme I, addition of the side chain fragment, sulfone (21), to the aldehyde (4), does not cause epimerization at the asymmetric center of carbon 20, i.e. the stereochemistry at that center is retained, as is required. If desired, retention of stereochemistry at carbon 20 may be checked at this stage of the synthesis by the conversion of intermediates of type (6) back to the original aldehyde starting materials. For example, subjecting compound (6) to ozonolysis with reductive work-up, using fully conventional and standard conditions, yields the corresponding C-22-aldehyde, i.e. the aldehyde of structure (4). Spectroscopic and chromatographic comparison of the aldehyde obtained from ozonolysis with the original starting material confirms retention of C-20 stereochemistry.

The next operation of the process involves conversion of these ring B-protected steroids to the desired 5,7-diene intermediate (7). In the case of the PTAD-diene-protected compound (6), this conversion is accomplished in a single step, namely treatment of (6) with a strong hydride reducing agent (e.g. LiAlH₄) in an ether solvent at reflux temperature gives the diene (7). The diene (7) is then converted to its 25-hydroxylated form (8) by known procedures in accordance with Scheme I.

Conversion of 5,7-diene (8) to the final vitamin D products (10) or (15) comprises a sequence of several steps. The sequence shown in Process Scheme I involves first the irradiation of an ether or hydrocarbon solution of the 5,7-diene (8) with ultraviolet light to yield the previtamin analog (9) which by warming (50°-90°C) in a suitable solvent (e.g. ethanol, hexane) undergoes isomerization to the 25-hydroxyvitamin D₂ compound (10).

Thereafter, compound (10) may be converted to the 1,25-dihydroxyvitamin D₂ compound (15) by the known steps shown in Scheme I. Reference is made to U. S. Patent Nos. 4,260,549 and 4,554,106 for the prior art relevant to these transformations.

The side chain fragment, sulfone (21), as used in Scheme I is specifically the (R) enantiomer. Therefore, compounds (10) or (15) are obtained as the C-24-R-epimers, 25-hydroxy-24-epi-vitamin D₂ (10) or 1,25-dihydroxy-24-epi-vitamin D₂ (15), respectively. Thus, compound (10) or (15) are prepared in epimerically pure form, and C-24-epimer separation as required in the process disclosed in U. S. atent No. 4,448,721, is not necessary. Use of the (S)-epimer of sulfone (21) in the present process yields specifically 25-OH-D₂, as well as, of course, the respective 1,25-dihydroxyvitamin D₂ compound.

The 5,7-diene (7) may be used as the free hydroxy compound or as its hydroxy-protected form, where the hydroxy-protecting groups (at C-3 and/or C-25) may be acyl, alkylsilyl or alkoxyalkyl groups as previously defined. Thus, the 25-OH-D₂ product will be obtained as the free hydroxy compound or, if desired, as the C-3, or C-25-hydroxy-protected, or 3,25-dihydroxy-protected derivatives. Synthesis according to Scheme I would provide the 25-OH-D₂ products as the free hydroxy compounds but analogous conversion of 5,7-diene intermediate (7) as the 3-, or 25-protected, or 3,25-di-protected derivative will yield the corresponding hydroxy-protected derivatives of the 25-OH-D₂ products.

The individual 25-OH-D₂ epimers, i.e. 25-OH-D₂ or 25-OH-24-epi-D₂ (10) when obtained in the free hydroxy forms, are also conveniently hydroxy-protected at the C-3 or C-25, or at both positions, by conventional reactions known in the art. Thus, 25-OH-D₂ may be acylated to yield, for example, the 25-OH-D₂-3-acetate, or the corresponding 3,25-diacetate. The 3-monoacetate, in a like fashion, may be further acylated at C-25 by treatment with a different acylating reagent, or, alternatively, the 3,25-diacetate may be selectively hydrolyzed by a mild base (KOH/MeOH) to give the 25-monoacetate, which if desired can be reacylated with a different acyl group at C-3. Other hydroxy-protecting groups can be introduced by analogous known reactions.

In addition to the hydroxy-protected derivatives, the 5,6-trans-isomers of 25-OH-24-epi-D₂ as well as the 1,25-dihydroxy compounds are compounds of potential utility in medical applications because of their considerable vitamin D-like activity. These 5,6-trans-compounds are prepared from the 5,6-cis-isomers (i.e. 10 or 15) by iodine catalyzed isomerization according to the procedures of Verloop et al. Rec. Trav. Chim. Pays Bas 78, 1004 (1969), and the corresponding 3- and/or 25-hydroxy-protected derivatives are likewise obtained by analogous isomerization of the corresponding 5,6-cis-acylates, or by hydroxy-protection of the 5,6-trans-25-OH-D₂ compounds.

The required side chain fragment, sulfone (21), can itself be prepared according to the process shown in Process Scheme II. This synthesis is straight-forward and involves as a first step the reaction of ester (16) in anhydrous tetrahydrofuran (THF) with methyl magnesium bromide to give the diol (17). Diol (17) is dissolved in anhydrous pyridine and reacted with p-toluenesulfonyl chloride to provide the tosylate (18). Tosylate (18) is dissolved in a solution of anhydrous dimethyl formamide and reacted with thiophenol and t-BuOK to yield the sulfide (19). The sulfide (19) in turn is dissolved in dichloromethane and reacted with 3-chloroperoxybenzoic acid to give the hydroxy sulfone compound (20). Pyridinium p-toluenesulfonate is then added to a solution of compound (20) in anhydrous dichloromethane and reacted with dihydropyran to yield the hydroxy protected tetrahydropyranyl sulfone (21a). The corresponding (S)-epimer of sulfone (21) can be prepared by the same process, using as starting material the ester corresponding to (16) but having the (S) configuration at carbon-2.

### Non-25-Hydroxylated Compounds (X₂ Is Hydrogen)

The reaction sequence illustrated by Process Scheme III presents another specific embodiment of the overall process, whereas Process Scheme IV illustrates preparation of an appropriate side chain unit for addition to the steroid-22-aldehyde as shown in Scheme III.

Starting materials for the present process are steroidal 22-aldehydes, such as, for example, the PTAD-diene-protected-22-aldehyde (4) shown in Scheme I (where PTAD refers to the phenyltriazoline-3,5-dione-protecting group shown), which in turn can be prepared from ergosterol by the known steps (Scheme I).

The first step of this process comprises the addition of a suitable side chain fragment. Thus, condensation of aldehyde (4) with a sulfonyl-side chain fragment as shown in the Scheme III (sulfone (35), further described below) present in the form or its anion, in an ether or hydrocarbon solvent, provides the hydroxy-sulfone intermediate (22). The anion of the sulfone (35) side chain fragment is generated by treatment of the sulfone with a strong base, such as lithium diethylamide, n-butyl lithium or methyl or ethyl magnesium bromide (or similar Grignard reagent) in an ether or hydrocarbon solvent, and to this solution of sulfone anion the steroid aldehyde (compound 4) as an ether or hydrocarbon solution is then added. The reaction is best effected under an inert atmosphere.

The next step comprises the removal of the hydroxy-and phenylsulfonyl groups in the side chain with formation of the 22(23)-trans-double bond. Thus, treatment of compound (22), in methanol solution saturated with NaHPO₄, with sodium amalgam under an inert atmosphere, gives compound (23) featuring the desired trans-22-double bond in the side chain. If desired, the 22-hydroxy group in compound (22) can also be acylated or sulfonylated (e.g. mesylated) prior to the Na/Hg-reduction step, but this is not generally required.

It is to be noted that, aid shown in Process Scheme III, addition of the side chain fragment, sulfone (35), to the aldehyde (4), does not cause epimerization at the asymmetric center of carbon 20, i.e. the stereochemistry at that center is retained, as is required. If desired, retention of stereochemistry at carbon 20 may be checked at this stage of the synthesis by the conversion of intermediates of type (23) back to the original aldehyde starting materials. For example, subjecting compound (23) to ozonolysis with reductive work-up, using fully conventional and standard conditions, yields the corresponding C-22-aldehyde, i.e. the aldehyde of structure (4). Spectroscopic and chromatographic comparison of the aldehyde obtained from ozonolysis with the original starting material confirms retention of C-20 stereochemistry.

The next operation of the process involves conversion of these ring B-protected steroids to the desired 5,7-diene intermediate (24). In the case of the PTAD-diene-protected compound (23), this conversion is accomplished in a single step, namely treatment of (23) with a strong hydride reducing agent (e.g. LiA1H₄) in an ether solvent at reflux temperature gives the diene (24).

Conversion of 5,7-diene (24) to the final vitamin D products (26) or (31) comprises a sequence of several steps. The sequence shown in Process Scheme III involves first the irradiation of an ether or hydrocarbon solution of the 5,7-diene (24) with ultraviolet light to yield the previtamin analog (25) which by warming (50°-90°C) in a suitable solvent (e.g. ethanol, hexane) undergoes isomerization to the vitamin D₂ compound (26).

Thereafter, compound (26) may be converted to the 1α-hydroxyvitamin D₂ compound (31) by the known steps shown in Scheme III. Reference is made to U. S. Patent Nos. 4,260,549 and 4,554,106 for the prior art relevant to these transformations.

The side chain fragment, sulfone (35), as used in Scheme III is specifically the (R) enantiomer. Therefore, compounds (26) or (31) are obtained as the C-24-R-epimers, 24-epi-vitamin D₂ (26) or 1α-hydroxy-24-epi-vitamin D₂ (31), respectively. Thus, compounds (26) or (31) are prepared in epimerically pure form, and C-24-epimer separation as required in the process disclosed in U. S. patent No. 4,448,721, is not necessary. Use of the (S)-epimer of sulfone (35) in the present process yields specifically vitamin D₂, as well as, of course, the respective 1α-hydroxyvitamin D₂ compound.

The 5,7-diene (24) may be used as the free hydroxy compound or as its hydroxy-protected form, where the hydroxy-protecting groups (at C-3) may be acyl, alkylsilyl or alkoxyalkyl groups as previously defined. Thus, the vitamin D₂ product will be obtained as the free hydroxy compound or, if desired, as the C-3-hydroxy-protected derivatives. Synthesis according to Scheme III would provide the vitamin D₂ products as the free hydroxy compounds but analogous conversion of 5,7-diene intermediate (24) as the 3-protected, derivative will yield the corresponding hydroxy-protected derivatives of the vitamin D₂ products.

The individual vitamin D₂ epimers, i.e. vitamin D₂ or 24-epi-D₂ (26) when obtained in the free hydroxy forms, are also conveniently hydroxy-protected at the C-3 position, by conventional reactions known in the art. Thus, vitamin D₂ may be acylated to yield, for example, the vitamin D₂-3-acetate. Other hydroxy-protecting groups can be introduced by analogous known reactions.

In addition to the hydroxy-protected derivatives, the 5,6-trans-isomers of 24-epi-D₂ as well as the 1α-hydroxy compounds are compounds of potential utility in medical applications because of their considerable vitamin D-like activity. These 5,6-trans-compounds can be prepared from the 5,6-cis-isomers (i.e. 26 or 31) by iodine catalyzed isomerization according to the procedures of Verloop et al. Rec. Trav. Chim. Pays Bas 78, 1004 (1969), and the corresponding 3-hydroxy-protected derivatives are likewise obtained by analogous isomerization of the corresponding 5,6-cis-acylates, or by hydroxy-protection of the 5,6-trans-D₂ compounds.

The required side chain fragment, sulfone (35), can be prepared according to Perlman et al, supra, or according to the process shown in Process Scheme IV. This synthesis is straightforward and involves as a first step dissolving alcohol (32) in anhydrous pyridine and reacting it with p-toluenesulfonyl chloride to provide the tosylate (33). Tosylate (33) is dissolved in a solution of anhydrous dimethyl formamide and reacted with thiophenol and t-BuOK to yield the sulfide (34). The sulfide (34) in turn is dissolved in dichloromethane and reacted with 3-chloroperoxybenzoic acid to give the sulfone compound (35). The corresponding (S)-epimer of sulfone (35) can also be prepared according to Perlman et al supra, or according to Process Scheme IV, using as starting material the alcohol corresponding to (32) but having the (S) configuration at carbon-2.

### Analog Compounds

Furthermore, the present process also serves as a convenient method for the synthesis of vitamin D₂ analogs (where X₂ is hydrogen) including 25-OH-D₂ analogs (where X₂ is hydroxy), of the formula (40) shown below, or of the corresponding 1α-hydroxy-analogs, 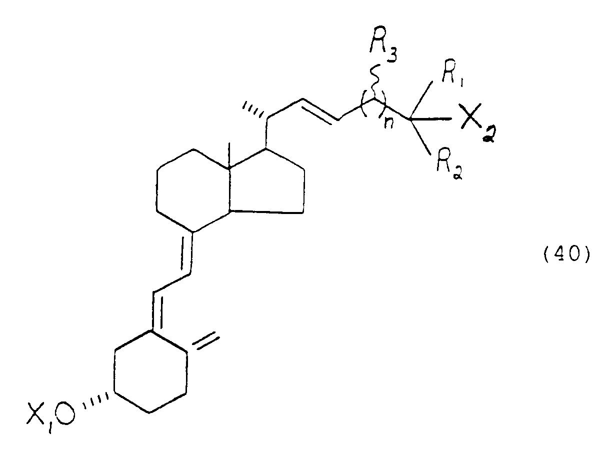
where n is an integer having a value of from 1 to 5, X₁ is selected from hydrogen and a hydroxy-protecting group, X₂ is selected from hydrogen, hydroxy and protected hydroxy, R₃ is alkyl, hydroxy, protected hydroxy, hydrogen or fluorine, and where R₁ and R₂, which may be the same or different, is an alkyl group or an aryl group and where the C-24-methyl group has either the (R)- or the (S)-stereochemical orientation. These compounds can be prepared by condensing compound (4) with the appropriate alkyl or aryl side chain fragment as shown by the following formulae, 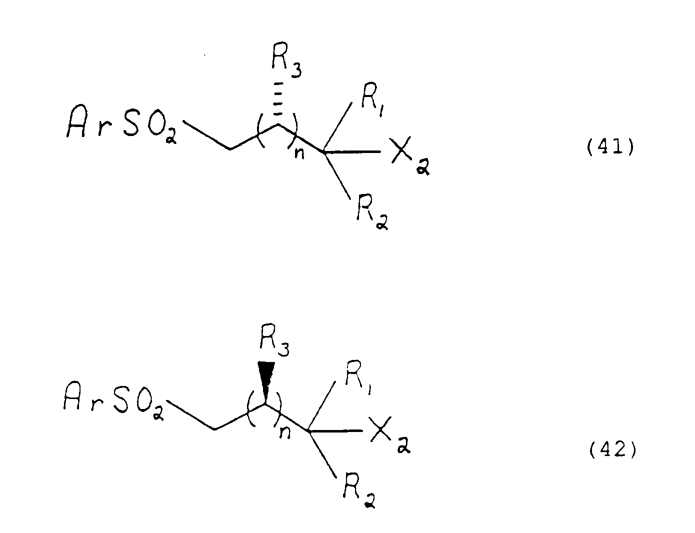
where X₂, R₁, R₂, R₃ and n are as defined above.

The use of compounds where (41) and (42) as side chain units in the synthetic process depicted as in Schemes I and III, then provides the 25-OH-D₂- or 25-OH-24-epi-D₂-homologs of general structure (40) where R₁, R₂ and R₃ represent alkyl or aryl residues or other substitutes as defined above. The products of general structure (40) can then be 1α-hydroxylated according to known methods (See U. S. Patent Nos. 4,260,549 and 4,554,106) so as to obtain the corresponding 1α-hydroxylated vitamin D homologs.

Since the compounds where R₁, R₂ or R₃ represent higher homologs of methyl, are generally more lipophilic, the alkyl- or aryl-analogues represented by structure (40) above or their 5,6-trans-isomers, are expected to have utility in applications where a greater degree of lipophilicity is desired.

WO 85/03939 discloses the compounds of the formula: 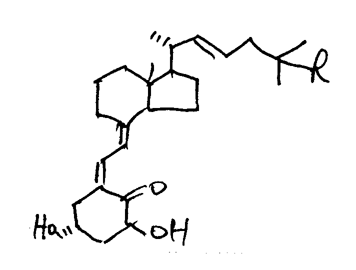
where R is hydrogen or hydroxy which can be obtained using a similar method
The present invention is further described by means of the following illustration. In this illustration, numerals designating specific products, e.g. compounds 1, 2, 3 etc. refer to the structures so numbered in Process Schemes I or II.

### EXAMPLE I

### Ergosterol Method

To a solution of 50g (0.13mol) of ergosterol 1 in 300ml of anhydrous pyridine was added 33.3ml (0.35mol) of acetic anhydride. The mixture was stirred at room temperature overnight and 600ml of water was added. The precipitate was filtered off, washed several times with 200ml of water and recrystallized from ethanol to obtain 2, 42.0g (76%). [yellowish crystal]

To a solution of 33g (0.075mol) of 2 in 500ml of chloroform was added 13.2g (0.075mol) of 4-phenyl-1,2, 4-triazoline-3,5-dione. The solution was stirred at room temperature for 30 min and 5ml of pyridine was added. The solution was cooled at -78°C and treated with an ozone-oxygen mixture for 30 min (TLC control) and thoroughly purged with nitrogen. 50ml of dimethyl sulfide was added and the mixture was washed with 300ml of water, 200ml of 2N HCl (twice) and 300ml of water. The organic layer was separated and each washing was extracted with 400ml and 200ml of chloroform. The combined extract was dried over Na₂SO₄ and concentrated in vacuo. The residue was purified on a silica gel column (5.06X63cm, 550g of 150^{∼} 425»m silica gel) using a mixture of ethyl acetate and hexane as eluant. 20.5g (50%) of 4 was eluted with 30% ethyl acetate in hexane. To increase yield, the recovered 3 (eluted with 15% ethyl acetate in hexane) was treated with an ozone-oxygen mixture as above.

To a stirred solution of 12.1g (37.1mmol) of sulfone 21, 5.10ml (36.4mmol) of diisopropyl amine and 100ml of anhydrous tetrahydrofuran (containing 1,10-phenanthroline as indicator) under nitrogen atmosphere at -78°C was added 22.7ml (36.3mml) of n-BuLi (1.6M in hexane). The solution was stirred under nitrogen at -78°C for 30min, then 10.0g (18.3mmol) of 4 in 40ml of anhydrous tetrahydrofuran was added. The mixture was stirred at -78°C for 1hr, decomposed by the addition of 100ml of saturated NH₄Cl solution, warmed to 0°C and extracted three times with 100ml of ethyl acetate. Each extract was washed with 100ml of saturated NaCl solution, dried over Na₂SO₄ and concentrated in vacuo. The residue was purified on a silica gel column (3.2X60cm, 150g of 75^{∼} 150»m silica gel). The unreacted sulfone 21 was eluted with benzene and 14.7g (92%) of 5 was eluted with ethyl acetate.

A mixture of 14.7g (16.9mmol) of hydroxysulfone 5, 110g of 5% sodium amalgum and 400ml of methanol saturated with Na₂HPO₄ was stirred under nitrogen atmosphere at 5°C for 20 hrs. The reaction solution was decanted and concentrated in vacuo. The residue was dissolved in 200ml of ethyl acetate and washed with 400ml and 200ml of water. The ethyl acetate extract was separated and each of washing was extracted twice with 200ml of ethyl acetate. The combined extract was dried over Na₂SO₄ and concentrated in vacuo. 10.5g (91%) of 6 was obtained.

To a solution of 10.5g (15.4mmol) of 6 in 400ml of tetrahydrofuran was added 11.5g (303.0mmol) of LiAlH₄. The mixture was heated under reflux and nitrogen atmosphere for 3 hrs, cooled with ice water and decomposed by the dropwise addition of 40ml of ethyl acetate and 60ml of water. Then, the mixture was filtered and the filtrate was concentrated in vacuo. The residue was dissolved in 200ml of ethyl acetate and washed twice with 200ml of saturated NaCl solution. The ethyl acetate extract was separated and each washing was extracted with 200ml of ethyl acetate. The combined extract was dried over Na₂SO₄ and concentrated in vacuo. Then, the residue was purified on a silica gel column (3.2X25cm, 80g of 75^{∼} 150»m silica gel) and 4.8g (63%) of 7 was eluted with 5% ether in benzene as eluant [yellow foam].

To a solution of 4.4g (8.9mmol) of 7 in 200ml of methanol and 130ml of dichloromethane was added 2.0g (7.9mmol) of pyridinium p-toluenesulfonate. The mixture was stirred at room temperature overnight, dissolved in 300ml of saturated NaCl solution and extracted three times with 400ml of dichloromethane. Each extract was washed with 400ml of saturated NaCl solution, combined, dried over Na₂SO₄ and concentrated in vacuo. The residue was recrystallized from ethanol to obtain 2.5g (68%) of 8 [white crystal]. To increase yield, the mother liquor was concentrated in vacuo, purified with chromatography and recrystallised from ethanol.

1.50g (3.6mmol) of 8 was dispersed in 500ml of a mixture of ether and benzene (4:1) and irradiated with stirring under nitrogen in a water-cooled quartz immersion well equipped with an ozone free filter using Eikosha's high-pressure UV lamp for 25min. The reaction was monitored by HPLC using Lichrosorb Si 60 (5 »m) column and 3% 2-propanol in hexane at 265nm.

The solution was concentrated in vacuo, redissolved in 100ml of ethanol and heated under reflux and nitrogen for 3 hrs. Then, the solution was concentrated in vacuo and the residue was purified on a silica gel column (3.2X50cm, 170g of 75^{∼} 150 »m of silica gel) using a mixture of ethyl acetate in hexane. 074g (50%) of 10 was eluted with 20% ethyl acetate in hexane. [white foam]
To a solution of 1.50g (3.6mmol) of 10 in 15ml of anhydrous pyridine was added 1.50g (7.9mmol) of tosyl chloride. The mixture was stirred under nitrogen at 5°C for 20 hrs. Then, the solution was poured into 200ml of cold saturated NaHCO₃ solution. The mixture was allowed to stand for 30 min and extracted three times with 150ml of a mixture of ether and dichloromethane (4:1). Each extract was washed with 150ml of saturated NaCl solution, 150ml of cold diluted HCl solution for twice, 150ml of saturated NaCl solution, 150ml of saturated NaHCO₃ solution and 150ml of saturated NaCl solution. The combined extract was dried over Na₂SO₄ and concentrated in vacuo. 1.90g (92%) of 11 was obtained and converted to 12 without further purification. [white foam]
4.40g of anhydrous KHCO₃ was dissolved in 200ml of anhydrous methanol under nitrogen at 50°C. To this solution was added dropwise a solution of 1.90g (3.4mmol) of 11 in 30ml of anhydrous dichloromethane. The mixture was stirred under nitrogen at 50°C for 21 hrs. Then, the solution was concentrated in vacuo, the residue was dissolved in 200ml of a mixture of ether and dichloromethane (4:1) and washed twice with 100ml of water. The organic extract was separated and each washing was extracted twice with 100ml of the same mixture of ether and dichloromethane. The combined extract was dried over Na₂SO₄ and concentrated in vacuo to obtain 1.50g (105%) of 12 which was hydroxylated without further purification. [yellow oil]
2.7ml (8.1mmol) of tert-butyl hydroperoxide (3.0M in 2,2-4-trimethylpentane) was added to a suspension of 220mg (2.0mmol) of selenium dioxide in 75ml of anhydrous dichloromethane. The mixture was stirred at room temperature under nitrogen for 30min. 0.3ml of anhydrous pyridine was added followed by a solution of 1.50g (3.5mmol) of 12 in 30ml of anhydrous dichloromethane. The mixture was stirred under nitrogen at room temperature for 30min and heated under reflux for 10min. Then, 50ml of 10% NaOH solution was added and the mixture was extracted with 200, 100 and 100ml of ether. Each extract was washed with 50ml of 10% NaOH solution and 50ml of saturated NaCl solution. The combined extract was dried over Na₂SO₄ and concentrated in vacuo. The residue was purified on a silica gel column (3.2cmX15cm, 50g of 75^{∼} 150 »m silica gel) using a mixture of ethyl acetate in hexane as eluant. 581mg (37%) of 13 was eluted with 30% ethyl acetate in hexane. [yellow oil]
581mg (1.3mmol) of 13 was dissolved in 5ml of acetic acid and heated at 50°C under nitrogen for 1 hr. Then, the solution was poured over ice and neutralized with 100ml of saturated NaHCO₃ solution. The mixture was extracted three times with 150ml of a mixture of ether and dichlormethane (4:1) Each extract was washed with 100ml of saturated NaHCO₃ solution and 100ml of saturated NaCl solution. The combined extract was dried over Na₂SO₄ and concentrated in vacuo. Then, to a solution of the residue in 10ml of ethyl acetate was added 120mg of maleic anhydride and the mixture was allowed to stand under nitrogen at room temperature for 2 hrs. Then, the solution was concentrated in vacuo, the residue was redissolved in 50ml of ether. 50ml of 0.1N KOH in methanol was added, the solution was stirred at room temperature for 1.5 hrs. and concentrated in vacuo. The residue was dissolved in 100ml of a mixture of ether and dichloromethane (4:1) and washed with 50ml of 10% NaOH solution for twice and 50ml of saturated NaCl solution. The organic extract was separated and each of washing was extracted twice with 100ml of the same mixture of ether and dichloromethane. The combined extract was dried over Na₂SO₄ and concentrated in vacuo. The residue was purified on a silica gel column (2.3X8.0cm, 10g of 45^{∼} 75 »m silica gel) using a mixture of ethyl acetate in hexane as eluant. 310 mg of 15 was eluted with 30% ethyl acetate in hexane, combined with another 337mg of 15 and recrystallized from methyl formate.

### EXAMPLE 2

### Intermediates for Side Chain

20g (0.169mol) of methyl (R)-(-)-3-hydroxy-2-methyl propionate 16 was dissolved in 60ml of anhydrous tetrahydrofuran and added under nitrogen atmosphere and ice cooling to a stirred solution of 245ml (0.735mol) of 3.0mol/1 methyl magnesium bromide in ether. At the end of the addition 100ml of anhydrous tetrahydrofuran was added to facilitate stirring. The mixture was stirred at room temperature for 2 hrs, decomposed by the careful addition of 150ml of 5N HCl with ice cooling and extracted three times with 200ml of ether. Each extract was washed with 150ml of saturated NaCl solution, combined and dried over Na₂SO₄. Evaporation afforded 16.4g (82%) of 17 as yellow oil.

A mixture of 16.4g (0.139mol) of 17, 26.5g (0.139mol) of tosyl chloride and 30ml of pyridine was stirred at 4°C overnight. Then, the reaction mixture was dissolved in 300ml of ether and washed with 200ml of water, 200ml of diluted HCl, 200ml of water and 200ml of saturated NaHCO₃ solution. The ether extract was separated and each washing was extracted twice with 200ml of ether. The combined extract was dried over Na₂SO₄ and concentrated in vacuo. The residue was purified on a silica gel column (5.5X20cm, 200g of 150 425m silica gel) and 32.1g (85%) of tosylate (18) was eluted with 10^{∼}20% ethyl acetate in hexane. [red oil]

To a stirred solution of 14.4g (0.131mol) of thiophenol in 70ml of anhydrous dimethyl formamide 14.4g (0.131mol) of t-BuOK was added followed by 32.1g (0.118mol) of 18 in 90ml anhydrous dimethyl formamide. The mixture was stirred overnight, dissolved in 300ml of ice water and extracted with 300,200 and 200ml of ethyl acetate. Each extract was washed with 200ml saturated NaHCO₃ solution and water, combined, dried over Na₂SO₄ and concentrated in vacuo. 28.0g (113%, containing dimethyl formamide) of 19 was obtained and was oxidized without further purification. [red oil]

28.0 (0.118mol) of 19 was dissolved in 400ml of dichloromethane and cooled with ice water. To this solution was added 51.7g (0.300mol) of m-chloroperbenzoic acid slowly, the mixture was stirred at room temperature for 2 hrs. and filtered. The filtrate was washed with 300ml of saturated NaHCO₃ solution for twice, 300ml of saturated Na₂SO₃ solution for twice and 300ml of saturated NaHCO₃ solution. The organic phase was separated and each washing was extracted twice with 300ml of dichloromethane. The combined extract was dried over Na₂SO₄, concentrated in vacuo and recrystallized from a mixture of ethyl acetate in hexane to obtain 20 25.2g (88%). [white crystals]

To a stirred solution of 20g (0.083mol) of 20 in 50ml of dichloromethane was added 20ml (0.221mol) of freshly distilled 2,3-dihydropyran followed by 0.8g of pyridinium p-toluenesulfonate. The mixture was stirred at room temperature for 2 hrs. and washed twice with saturated NaCl solution. The organic phase was separated and each of washing was extracted twice with 50ml of dichloromethane. The combined extract was dried over Na₂SO₄ and concentrated in vacuo. The residue was purified on a silica gel column (3.2X45cm, 150g of 75 ^{∼} 150 »m silica gel) and 26.0g (96%) of 21a was eluted with benzene as eluant. [colorless oil]

### EXAMPLE 3

To a solution of ergosterol 1 in anhydrous pyridine is added acetic anhydride. The mixture is stirred at room temperature overnight and water is added. The precipitate is filtered off, washed several times with water and recrystallized from ethanol to obtain 2.

To a solution of the precipitate 2 in chloroform is added 4-phenyl-1, 2, 4-triazoline-3,5-dione. The solution is stirred at room temperature and pyridine is added. The solution is cooled and treated with an ozone-oxygen mixture (TLC control) and thoroughly purged with nitrogen. Dimethyl sulfide is added and the mixture is washed with water, 2N HC1 and then water again. The organic layer is separated and each washing is extracted with chloroform. The combined extract is dried over Na₂SO₄ and concentrated in vacuo. The residue is purified by silica gel chromatography to obtain compound 4.

To a stirred solution of sulfone 35, diisopropyl amine and anhydrous tetrahydrofuran (containing 1,10-phenanthroline as indicator) under nitrogen atmosphere is added n-BuLi (1.6M in hexane). The solution is stirred under nitrogen, then, compound 4 in anhydrous tetrahydrofuran is added. The mixture is stirred, decomposed by the addition of saturated NH₄Cl solution, warmed and extracted three times with ethyl acetate. Each extract is washed with saturated NaCl solution, dried over Na₂SO₄ and concentrated in vacuo. The residue is purified on a silica gel column to afford compound 22.

A mixture of hydroxysulfones 22, 5% sodium amalgum and methanol saturated with Na₂HPO₄ is stirred under nitrogen atmosphere. The reaction solution is decanted and concentrated in vacuo. The residue is dissolved in ethyl acetate and washed with water. The ethyl acetate extract is separated and each washing is extracted twice with ethyl acetate. The combined extract is dried over Na₂SO₄ and concentrated in vacuo to obtain 23.

To a solution of compound 23 in tetrahydrofuran is added LiAlH₄. The mixture is heated under reflux and nitrogen atmosphere, cooled with ice water and decomposed by the dropwise addition of ethyl acetate and water. Then, the mixture is filtered and the filtrate is concentrated in vacuo. The residue is dissolved in ethyl acetate and washed twice with saturated NaCl solution. The ethyl acetate extract is separated and each washing is extracted with ethyl acetate. The combined extract is dried over Na₂SO₄ and concentrated in vacuo. Then, the residue is purified on a silica gel column to provide compound 24.

Compound 24 is dispersed in a mixture of ether and benzene (4:1) and irradiated with stirring under nitrogen in a water-cooled quartz immersion well equipped with an ozone free filter using a high-pressure UV lamp. The reaction may be monitored by HPLC.

The solution is concentrated in vacuo, redissolved in ethanol and heated under reflux and nitrogen. Then, the solution is concentrated in vacuo and the residue is purified on a silica gel column to obtain compound 26.

To a solution of compound 26 in anhydrous pyridine is added tosyl chloride. The mixture is stirred under nitrogen. Then, the solution is poured into a cold saturated NaHCO₃ solution. The mixture is allowed to stand for 30min and extracted three times with a mixture of ether and dichloromethane (4:1). Each extract is washed with saturated NaCl solution, cold diluted HCl solution twice, saturated NaCl solution, saturated NaHCO₃ solution and saturated NaCl solution. The combined extract is dried over Na₂SO₄ and concentrated in vacuo. Compound 27 is obtained and converted to 28 without further purification.

Anhydrous KHCO₃ is dissolved in anhydrous methanol under nitrogen. To this solution is added dropwise a solution of compound 27 in anhydrous dichloromethane. The mixture is stirred under nitrogen. Then, the solution is concentrated in vacuo, the residue is dissolved in a mixture of ether and dichloromethane (4:1) and washed twice with water. The organic extract is separated and each washing is extracted twice with the same mixture of ether and dichloromethane. The combined extract is dried over Na₂SO₄ and concentrated in vacuo to obtain Compound 28 which is hydroxylated without further purification.

Tert-butyl hydroperoxide (3.0M in 2,2-4-trimethylpentane) is added to a suspension of selenium dioxide in anhydrous dichloromethane. The mixture is stirred at room temperature under nitrogen. Anhydrous pyridine is added followed by a solution of compound 28 in anhydrous dichloromethane. The mixture is stirred under nitrogen at room temperature and heated under reflux. Then, a 10% NaOH solution is added and the mixture is extracted with ether. Each extract is washed with a 10% NaOH solution and a saturated NaCl solution. The combined extract is dried over Na₂SO₄ and concentrated in vacuo. The residue is purified on a silica gel column to obtain compound 29.

Compound 29 is dissolved in acetic acid and heated under nitrogen. Then, the solution is poured over ice and neutralized with saturated NaHCO₃ solution. The mixture is extracted three times with a mixture of ether and dichloromethane (4:1). Each extract is washed with a saturated NaHCO₃ solution and a saturated NaCl solution. The combined extract is dried over Na₂SO₄ and concentrated in vacuo. Then, to a solution of the residue in ethyl acetate is added maleic anhydride and the mixture is allowed to stand under nitrogen at room temperature. Then, the solution is concentrated in vacuo, the residue is redissolved in ether. KOH in methanol is added, the solution is stirred at room temperature and concentrated in vacuo. The residue is dissolved in a mixture of ether and dichloromethane (4:1) and washed with 10% NaOH solution twice and a saturated NaCl solution. The organic extract is separated and each washing is extracted twice with the same mixture of ether and dichloromethane. The combined extract is dried over Na₂SO₄ and concentrated in vacuo. The residue is purified on a silica gel column using a mixture of ethyl acetate in hexane as eluant to obtain compound 31.

### EXAMPLE 4

### Intermediates for Side Chain

A mixture of compound 32, tosyl chloride and pyridine is stirred overnight. Then, the reaction mixture is dissolved in ether and washed with water, diluted HC1, water and saturated NaHCO₃ solution. The ether extract is separated and each washing is extracted twice with ether. The combined extract is dried over Na₂SO₄ and concentrated in vacuo. The residue is purified on a silica gel column to give tosylate (33).

To a stirred solution of thiophenol in anhydrous dimethyl formamide t-BuOK is added followed by compound 33 in anhydrous dimethyl formamide. The mixture is stirred overnight, dissolved in ice water and extracted with ethyl acetate. Each extract is washed with a saturated NaHCO₃ solution and water, combined, dried over Na₂SO₄ and concentrated in vacuo. Compound 34 is obtained and oxidized without further purification.

Compound 34 is dissolved in dichloromethane and cooled with ice water. To this solution is added m-chloroperbenzoic acid slowly, the mixture is stirred at room temperature for 2 hrs. and filtered. The filtrate is washed with a saturated NaHCO₃ solution twice, a saturated Na₂SO₃ solution twice and a saturated NaHCO₃ solution. The organic phase is separated and each washing is extracted twice with dichloromethane. The combined extract is dried over Na₂SO₄, concentrated in vacuo and recrystallized from a mixture of ethyl acetate in hexane to form compound 35.

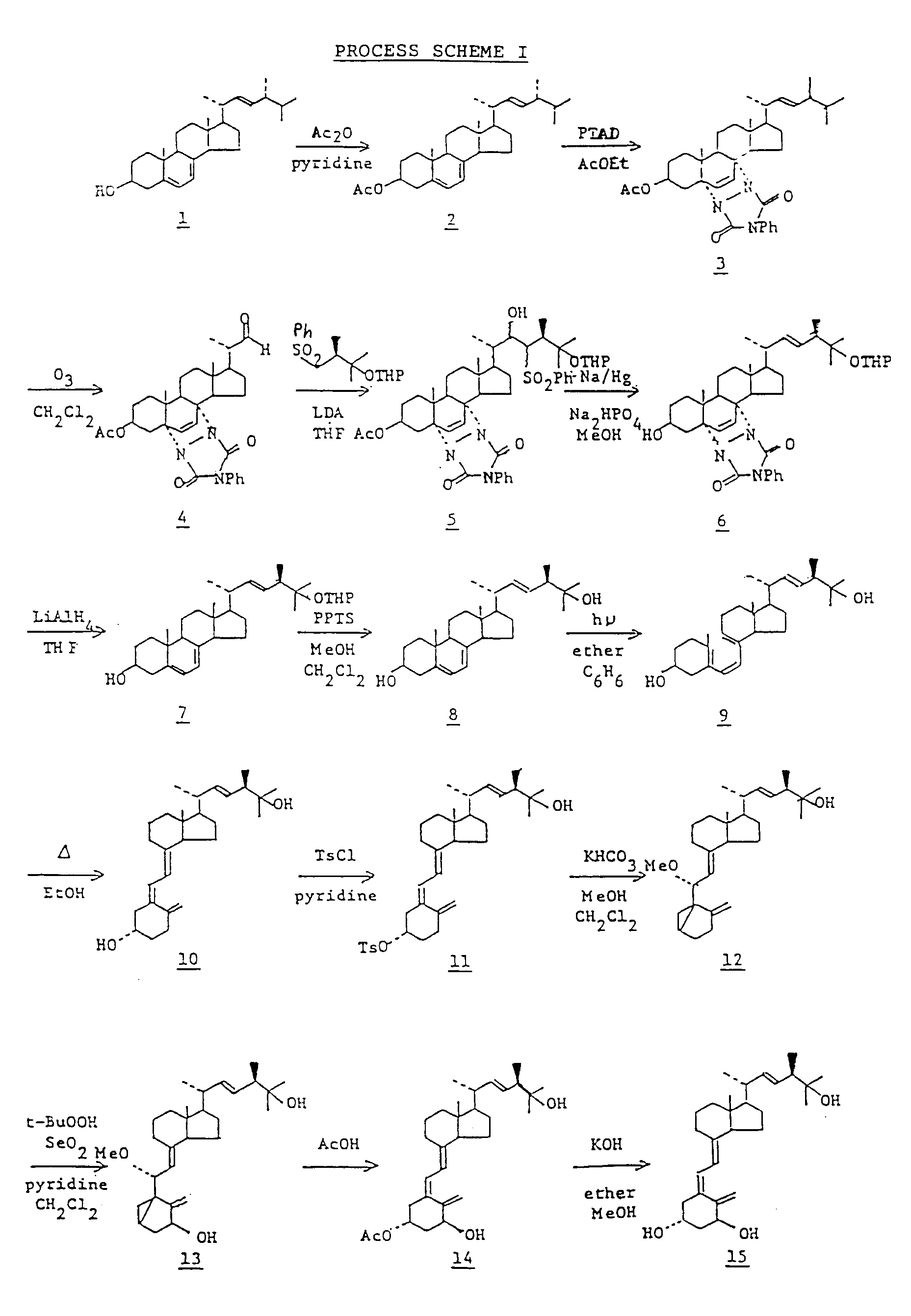

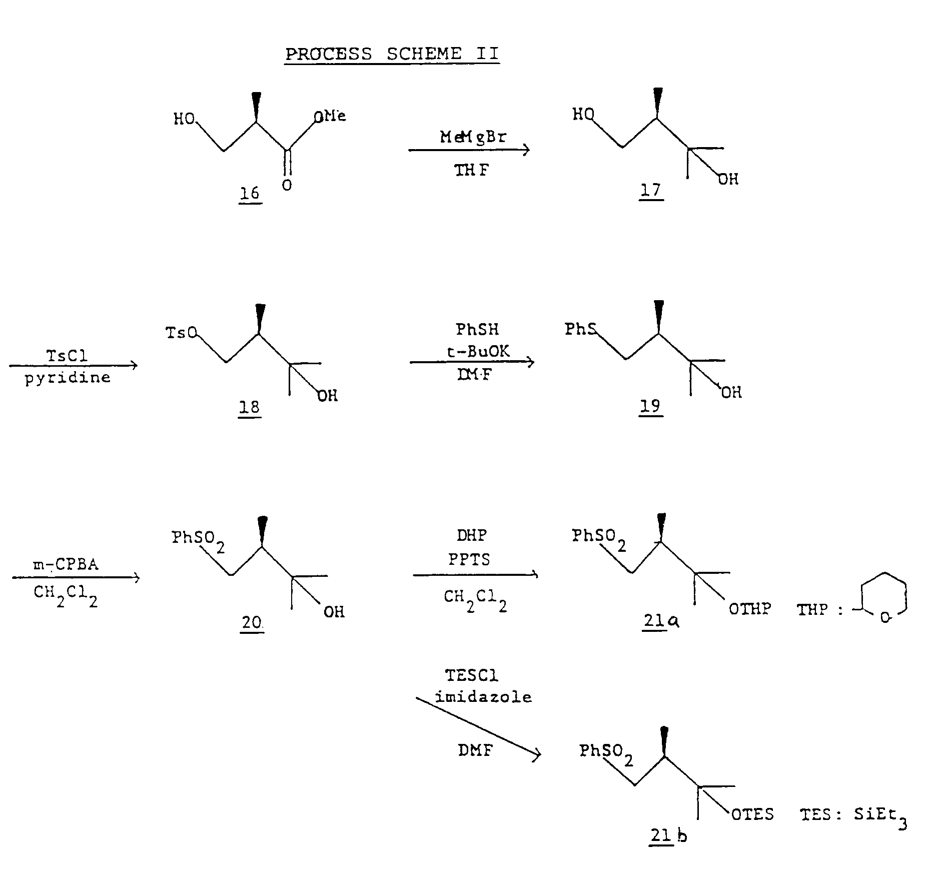

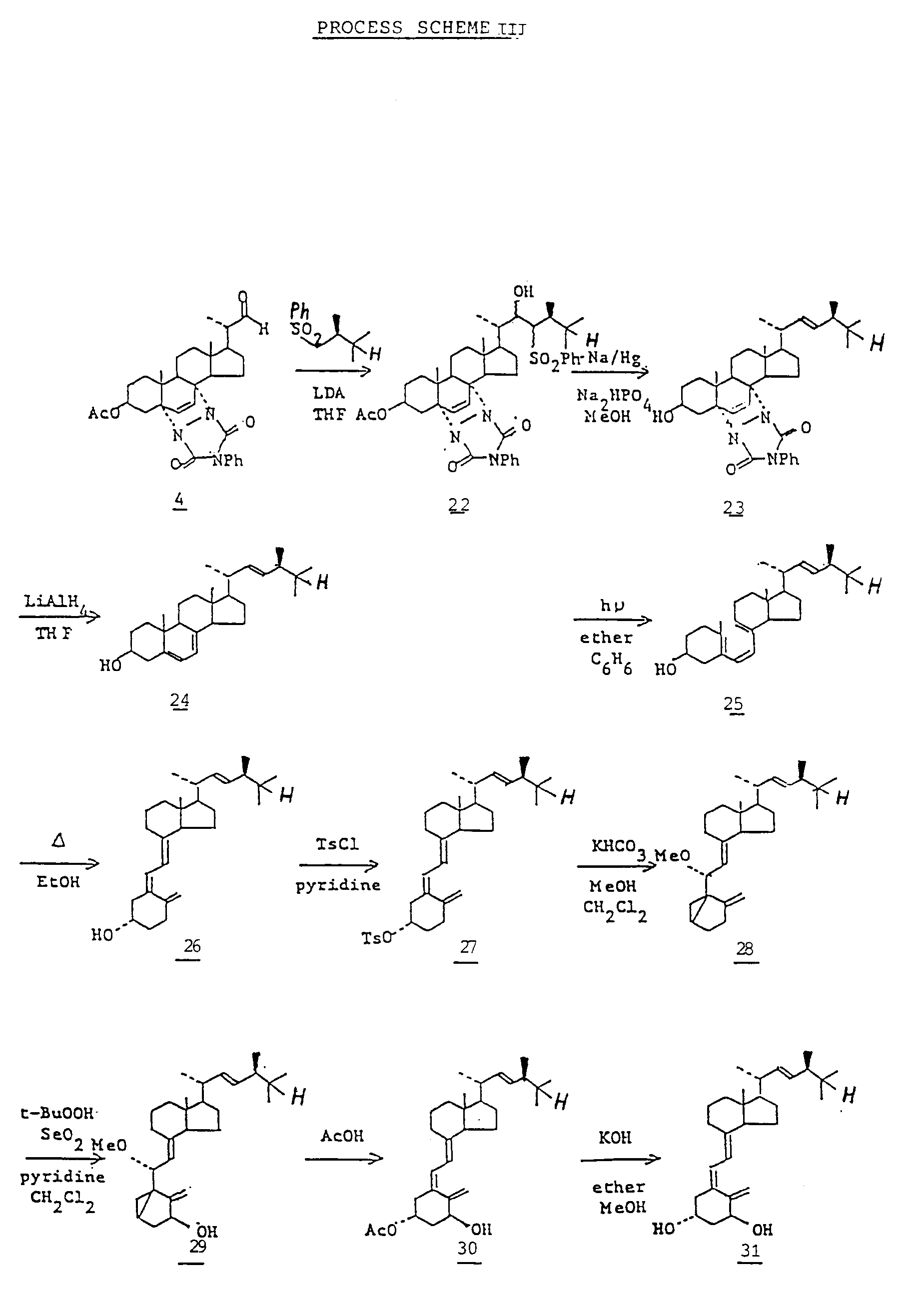

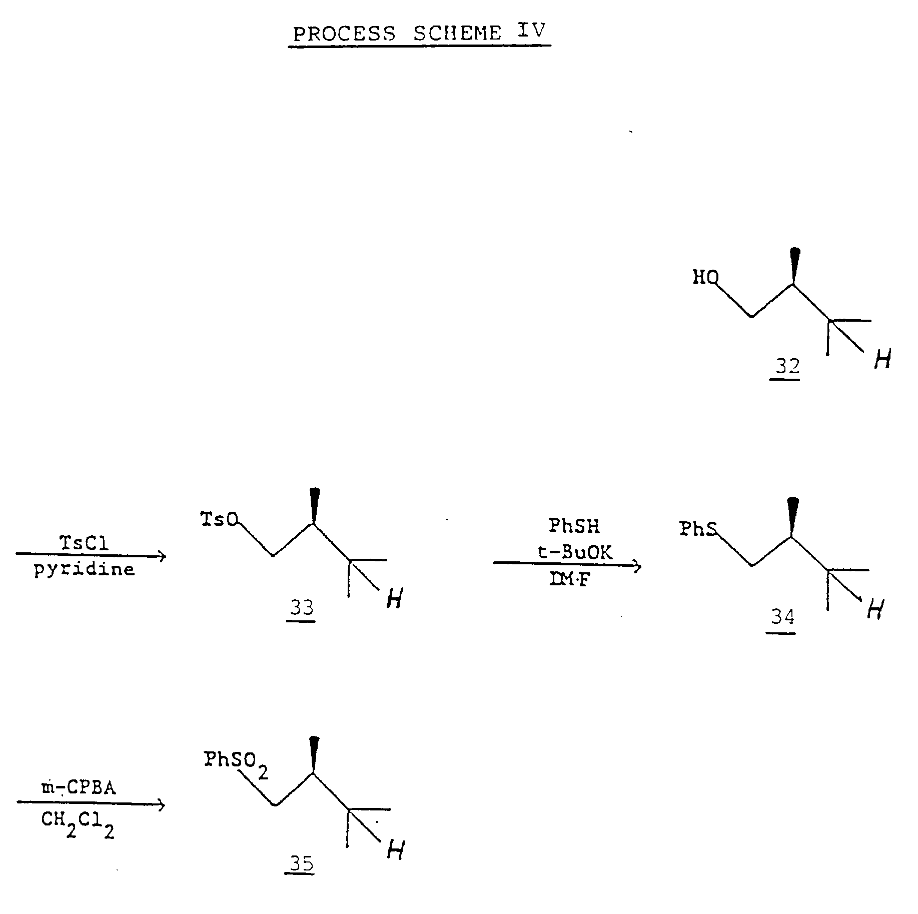

## Claims

1. A method for preparing a vitamin D compound having the formula 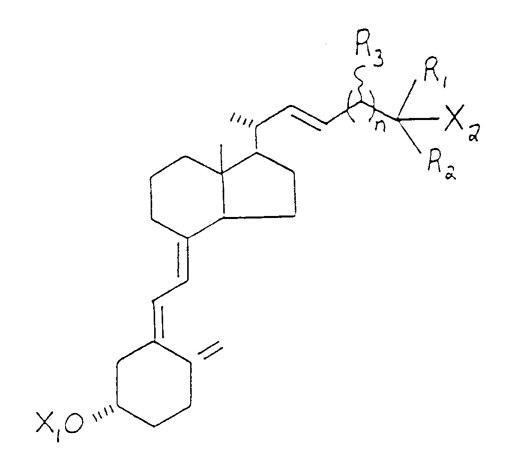 where R₃ may have the R or S configuration and wherein n is an integer having a value of from 1 to 5, X₁ is hydrogen or a hydroxy protecting group, X₂ is hydrogen, hydroxy, or protected hydroxy, R₃ is alkyl, hydroxy, protected hydroxy, hydrogen or fluorine, and wherein R₁ and R₂, which may be the same or different, are each an alkyl or aryl group, with the proviso that when n=1 and R₃ is hydrogen, R₁ and R₂ cannot both be methyl, which comprises condensing a steroidal aldehyde of the formula 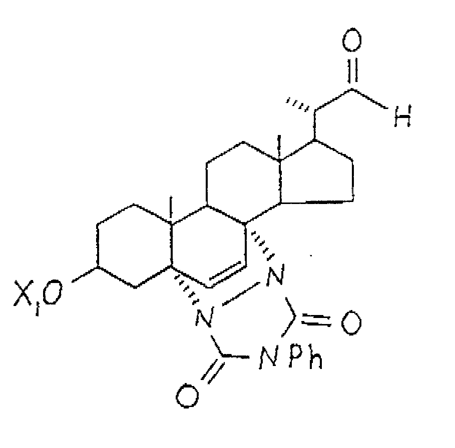 wherein X₁ is as defined above, with an arylsulfone of the formula
ArSO₂CH₂R
wherein Ar is aryl, R is alkyl, hydroxylated alkyl, hydroxy-protected hydroxylated alkyl, fluoro-substituted alkyl, fluoro-substituted hydroxylated alkyl or fluoro-substituted hydroxy-protected alkyl, wherein a hydroxy-sulfonyl adduct of the formula 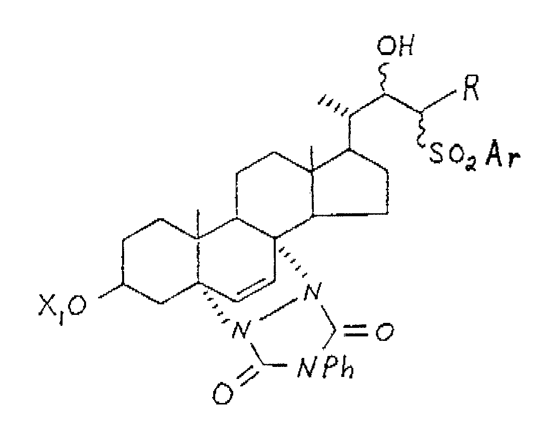 is obtained, wherein Ar, R and X₁ are as defined above, optionally acylating or sulfonylating the C-22-hydroxy group of said adduct, reducing said adduct to obtain an intermediate of the formula 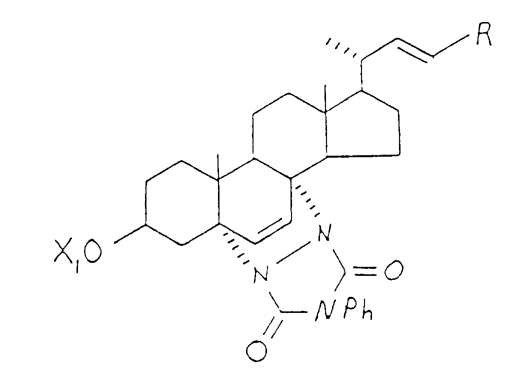 wherein R and X₁ are as defined above, and converting said intermediate to obtain the desired vitamin D compound, said converting step comprising removal of the triazoline group to form a 5,7-diene steroid intermediate, subjecting said intermediate to irradiation with ultraviolet light in an ether or hydrocarbon, isomerising the resulting previtamin D compound in a solvent at a temperature from 50° to 90°C and removing, or exchanging and then removing, any hydroxy protecting group.

2. The method of claim 1 wherein X₁ and X₂ are both hydrogen.

3. The method of claim 1 or 2 wherein R₁ and R₂ are both an alkyl group.

4. The method of claim 1 wherein X₁ is hydrogen and X₂ is hydroxy.

5. The method of claim 4 wherein R₁ and R₂ are both an alkyl group.

6. The method of claim 1 wherein the vitamin D compound is 25-hydroxy-24-epi-vitamin D₂.

7. The method of any one of the preceding claims wherein the desired vitamin D compound is further subjected to a 1α hydroxylation process so as to obtain the corresponding 1α-hydroxylated vitamin D compound.

8. A method for preparing 25-hydroxy-24-epi-vitamin D₂ having the formula 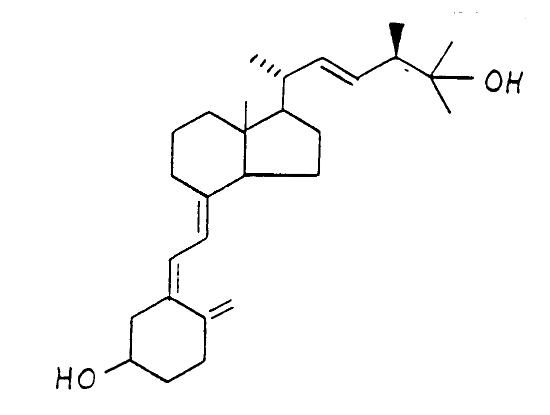 which comprises condensing a steroidal aldehyde of the formula: 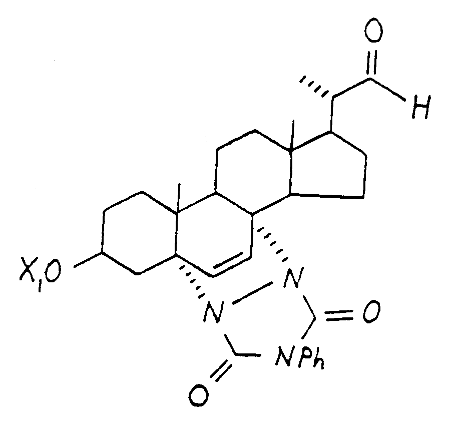 where X₁ is a hydroxy protecting group, with an arylsulfone of the formula 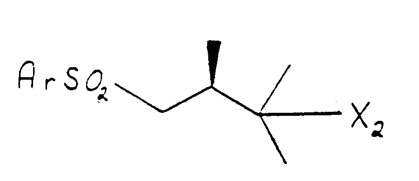 wherein X₂ is a protected hydroxy group whereby a hydroxy-sulfonyl adduct of the formula 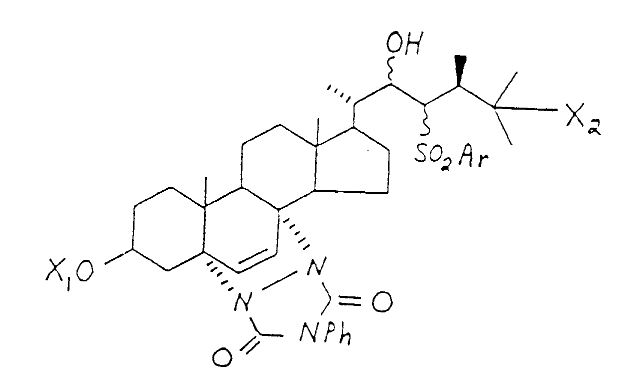 is obtained wherein X₁ and X₂ are as defined above, optionally acylating or sulfonylating the C-22-hydroxy group of said adduct, reducing said adduct to obtain an intermediate of the formula 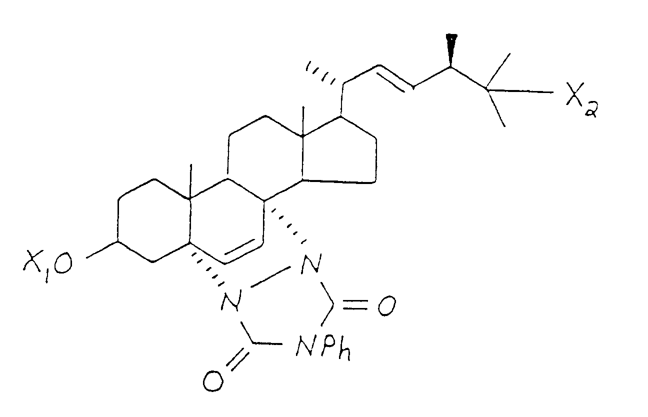 where X₁ is hydrogen or a hydroxy protecting group, and X₂ is a protected hydroxy group, and converting said intermediate to obtain 25-hydroxy-24-epi-vitamin D₂, said converting step comprising removal of the triazoline group to form a 5,7-diene steroid intermediate, subjecting said intermediate to irradiation with ultraviolet light in an ether or hydrocarbon, isomerising the resulting previtamin D compound in a solvent at a temperature from 50° to 90°C and removing, or exchanging and then removing, any hydroxy protecting group.

9. The method of claim 8 wherein 25-hydroxy-24-epi-vitamin D₂ is further subjected to a 1α hydroxylation process so as to obtain the corresponding 1α hydroxylated vitamin D compound.

10. The method of claim 9 wheein the compound is 1α,25-dihydroxy-24-epi-vitamin D₂.

## Patentansprüche

1. Verfahren zur Herstellung einer Vitamin-D-Verbindung der Formel 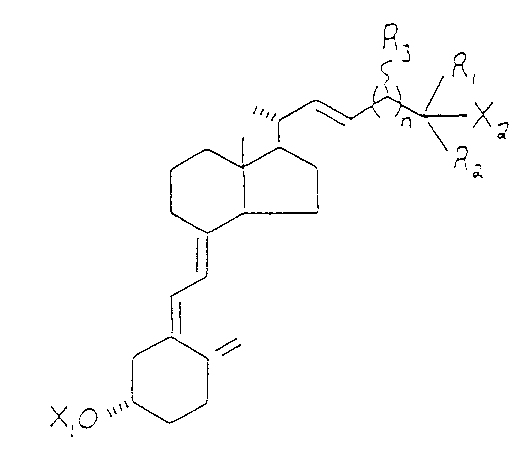 worin R3 die R oder S-Konfiguration besitzen kann, und worin n eine ganze Zahl mit einem Wert von 1 bis 5 ist, X1 Wasserstoff oder eine Hydroxyschutzgruppe ist, X2 Wasserstoff, Hydroxy oder geschütztes Hydroxy ist, R3 Alkyl, Hydroxy, geschütztes Hydroxy, Wasserstoff oder Fluor ist, und worin R1 und R2, die gleich oder verschieden sein können, eine Alkyl- oder Arylgruppe bedeuten, mit der Maßnahme, daß, wenn n = 1 und R3 Wasserstoff ist, R1 und R2 nicht beide Methyl sein können, dadurch gekennzeichnet, daß man einen Steroid-Aldehyd der Formel 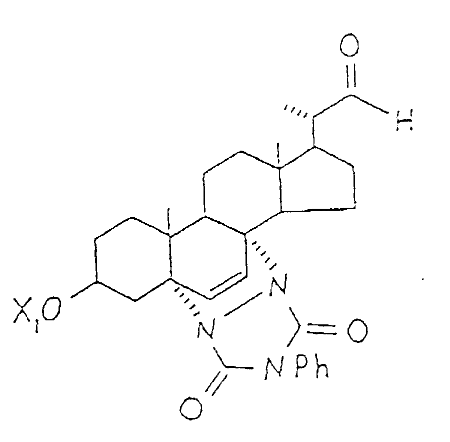 worin X1 die vorstehend angegebene Bedeutung besitzt, mit einem Arylsulfon der Formel
ArSO₂CH₂R
worin Ar Aryl ist, R Alkyl, hydroxyliertes Alkyl, hydroxyliertes Alkyl mit geschützter Hydroxygruppe, Fluor-substituiertes Alkyl, Fluor-substituiertes hydroxyliertes Alkyl oder Fluor-substituiertes Hydroxyalkyl mit geschützter Hydroxygruppe ist, kondensiert, wobei ein Hydroxy-Sulfonyl-Addukt der Formel 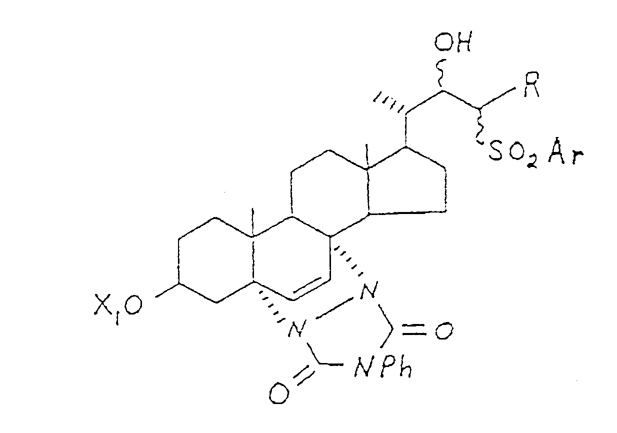 erhalten wird, worin Ar, R und X1 die vorstehend angegebene Bedeutung besitzen, gegebenenfalls die C-22-Hydroxygruppe des Addukts acyliert oder sulfoniert, das Addukt reduziert, um ein Zwischenprodukt der Formel 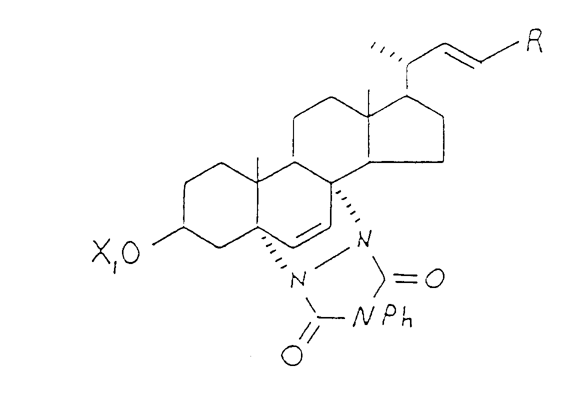 zu erhalten, worin R und X1 die vorstehend angegebene Bedeutung besitzen, und dieses Zwischenprodukt in die gewünschte Vitamin D-Verbindung überführt, wobei der Überführungsschritt umfaßt die Entfernung der Triazolingruppe zur Bildung eines 5,7-Diensteroid-Zwischenproduktes, Bestrahlen dieses Zwischenproduktes mit ultraviolettem Licht in einem Ether oder Kohlenwasserstoff, Isomerisieren der resultierenden Provitamin D-Verbindung in einem Lösungsmittel bei einer Temperatur von 50 bis 90 °C und Entfernen, oder Austausch und nachfolgendes Entfernen einer Hydroxyschutzgruppe.

2. Verfahren nach Anspruch 1, dadurch gekennzeichnet, daß X1 und X2 beide Wasserstoff sind.

3. Verfahren nach Anspruch 1 oder 2, dadurch gekennzeichnet, daß R1 und R2 beide eine Alkylgruppe sind.

4. Verfahren nach Anspruch 1, dadurch gekennzeichnet, daß X1 Wasserstoff und X2 Hydroxy ist.

5. Verfahren nach Anspruch 4, dadurch gekennzeichnet, daß R1 und R2 beide eine Alkylgruppe sind.

6. Verfahren nach Anspruch 1, dadurch gekennzeichnet, daß die Vitamin-D-Verbindung 25-Hydroxy-24-epi-Vitamin D2 ist.

7. Verfahren nach einem der vorhergehenden Ansprüche, dadurch gekennzeichnet, daß die gewünschte Vitamin-D-Verbindung ferner einem 1α-Hydroxylierungsverfahren unterworfen wird, um die entsprechende 1α-hydroxylierte Vitamin-D-Verbindung zu erhalten.

8. Verfahren zur Herstellung von 25-Hydroxy-24-epi-Vitamin D2 der Formel 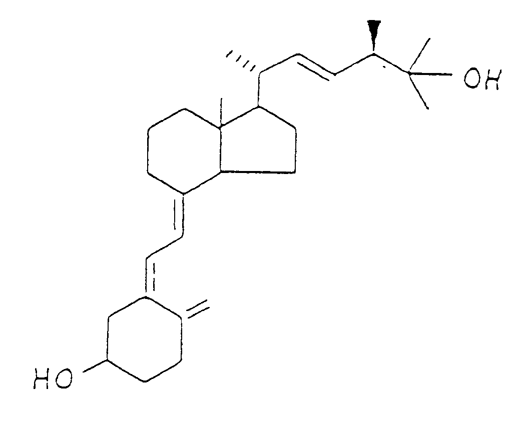 dadurch gekennzeichnet, daß man ein Steroid-Aldehyd der Formel: 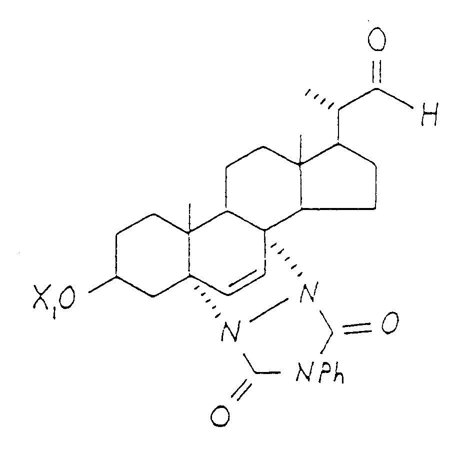 worin X1 eine Hydroxyschutzgruppe ist, mit einem Arylsulfon der Formel 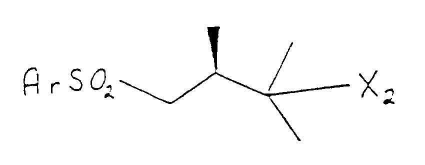 worin X2 eine geschützte Hydroxygruppe ist, kondensiert, wobei ein Hydroxy-Sulfonyl-Addukt der Formel 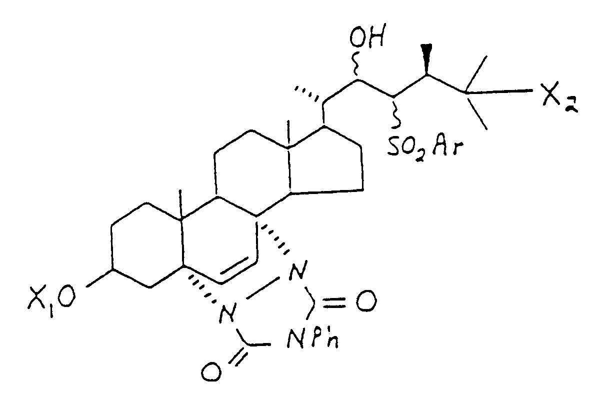 erhalten wird, worin X1 und X2 die vorstehend angegebene Bedeutung besitzen, die C-22-Hydroxygruppe gegebenenfalls acyliert oder sulfoniert, das Addukt reduziert um ein Zwischenprodukt der Formel 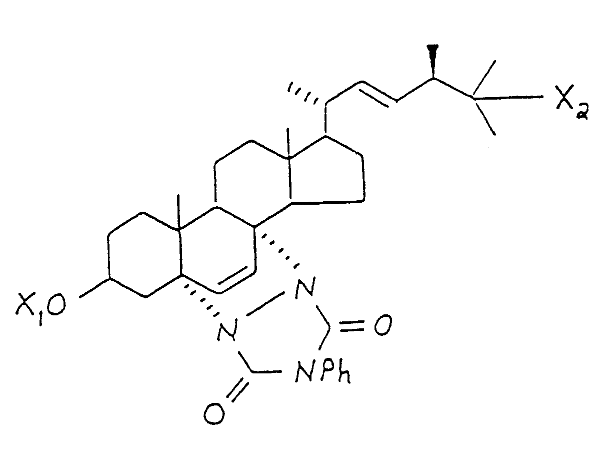 zu erhalten, worin X1 Wasserstoff oder eine Hydroxyschutzgruppe ist, und X2 eine geschützte Hydroxygruppe ist, und dieses Zwischenprodukt in ein 25-Hydroxy-24-epi-Vitamin D2 überführt, wobei der Überführungsschritt umfaßt die Entfernung der Triazolingruppe zur Bildung eines 5,7-Diensteroid-Zwischenprodukts, Bestrahlen dieses Zwischenproduktes mit ultraviolettem Licht in einem Ether oder Kohlenwasserstoff, Isomerisieren der resultierenden Provitamin D-Verbindung in einem Lösungsmittel bei einer Temperatur von 50 bis 90 °C und Entfernen, oder Austausch und nachfolgendes Entfernen einer Hydroxyschutzgruppe.

9. Verfahren nach Anspruch 8, dadurch gekennzeichnet, daß das 25-Hydroxy-24-epi-Vitamin D2 ferner einem 1α-Hydroxylierungsverfahren unterworfen wird, um die entsprechende 1α-hydroxylierte Vitamin-D-Verbindung zu erhalten.

10. Verfahren nach Anspruch 9, dadurch gekennzeichnet, daß die Verbindung 1α,25-Hydroxy-24-epi-Vitamin D2 ist.

## Revendications

1. Procédé de préparation d'un dérivé de vitamine D, répondant à la formule: 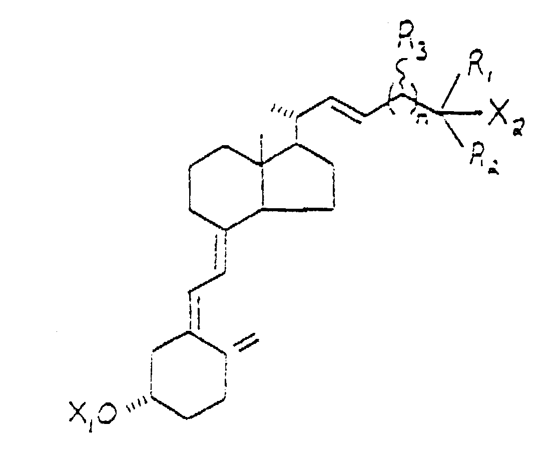 dans laquelle R₃ peut présenter la configuration R ou S, n représente un nombre entier ayant une valeur de 1 à 5, X₁ représente un atome d'hydrogène ou un groupe protégeant le groupe hydroxy, X₂ représente un atome d'hydrogène ou un groupe hydroxy ou hydroxy protégé, R₃ représente un groupe alkyle, hydroxy ou hydroxy protégé, ou un atome d'hydrogène ou de fluor, et R₁ et R₂, qui peuvent être identiques ou différents, représentent chacun un groupe alkyle ou aryle, étant entendu que, lorsque n est égal à 1 et R₃ représente un atome d'hydrogène, R₁ et R₂ ne peuvent pas représenter tous deux des groupes méthyle, procédé qui comprend la condensation d'un aldéhyde stéranique de formule: 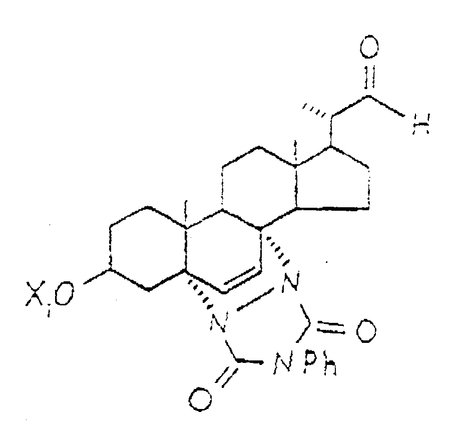 dans laquelle X₁ est tel que défini précédemment, avec une arylsulfone de formule:
ArSO₂CH₂R
dans laquelle Ar représente un groupe aryle, et R représente un groupe alkyle, alkyle hydroxylé, alkyle hydroxylé à groupe hydroxy protégé, fluoroalkyle, fluoroalkyle hydroxylé ou fluoroalkyle hydroxylé à groupe hydroxy protégé, ce qui permet d'obtenir un produit d'addition sulfonylé et hydroxylé, de formule: 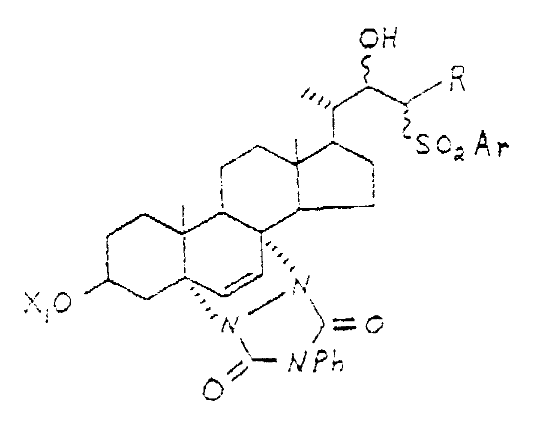 dans laquelle Ar, R et X₁ sont tels que définis précédemment,
l'acylation ou la sulfonylation éventuelle du groupe hydroxy du C-22 dudit produit d'addition,
la réduction dudit produit d'addition pour l'obtention d'un produit intermédiaire de formule: 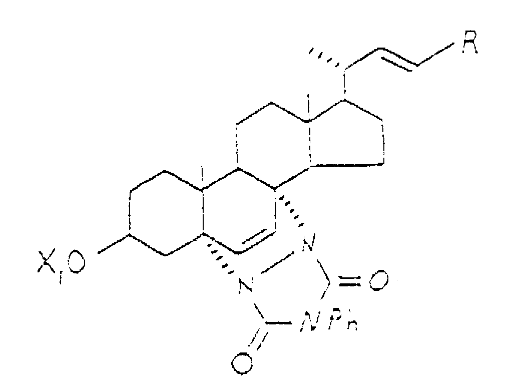 dans laquelle R et X₁ sont tels que définis précédemment,
et la transformation dudit produit intermédiaire pour l'obtention du dérivé désiré de vitamine D, ladite étape de transformation comprenant l'élimination du groupe triazoline de manière à former un produit intermédiaire stéranique 5,7-diénique, la soumission dudit produit intermédiaire, en solution dans un éther ou un hydrocarbure, à l'action des rayons ultraviolets, l'isomérisation du dérivé de prévitamine D résultant, dans un solvant, à une température de 50°C à 90°C, et l'élimination ou l'échange puis l'élimination de tout groupe protecteur de groupe hydroxy.

2. Procédé selon la revendication 1, dans lequel X₁ et X₂ représentent tous deux des atomes d'hydrogène.

3. Procédé selon la revendication 1 ou 2, dans lequel R₁ et R₂ représentent tous deux un groupe alkyle.

4. Procédé selon la revendication 1, dans lequel X₁ représente un atome d'hydrogène et X₂ représente un groupe hydroxy.

5. Procédé selon la revendication 4, dans lequel R₁ et R₂ représentent tous deux un groupe alkyle.

6. Procédé selon la revendication 1, dans lequel le dérivé de vitamine D est la 25-hydroxy-24-épi-vitamine D₂.

7. Procédé selon l'une quelconque des revendications précédentes, dans lequel on soumet en outre le dérivé désiré de vitamine D à un procédé d'hydroxylation en 1α, de façon à obtenir le dérivé de vitamine D 1α-hydroxylé correspondant.

8. Procédé de préparation de la 25-hydroxy-24-épi-vitamine D₂, répondant à la formule: 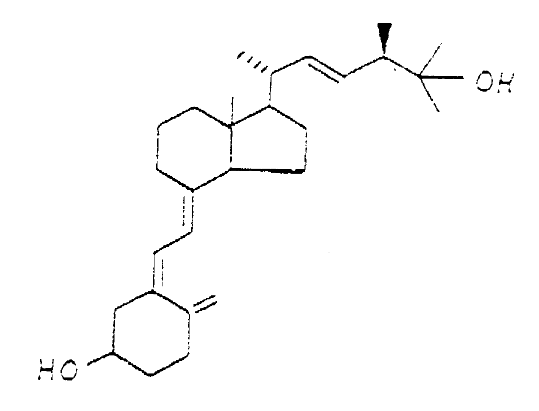 qui comprend la condensation d'un aldéhyde stéranique de formule: 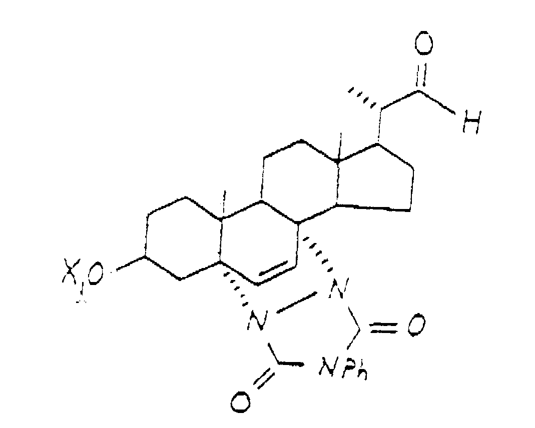 dans laquelle X₁ représente un groupe protégeant le groupe hydroxy, avec une arylsulfone de formule: 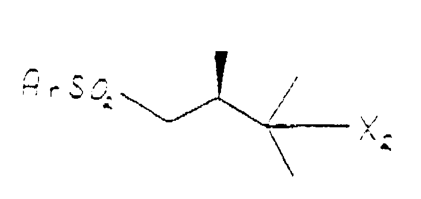 dans laquelle X₂ représente un groupe hydroxy protégé, ce qui permet d'obtenir un produit d'addition sulfonylé et hydroxylé, de formule: 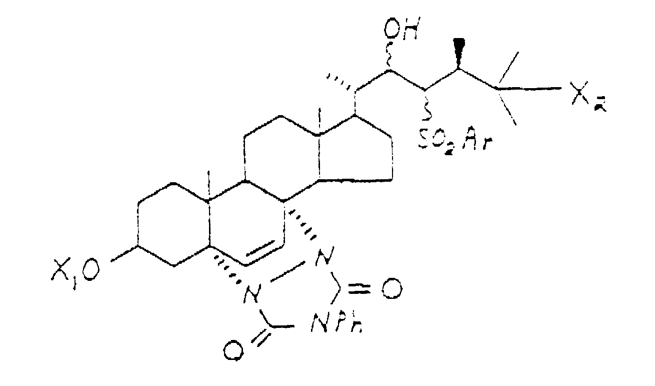 dans laquelle X₁ et X₂ sont tels que définis précédemment,
l'acylation ou la sulfonylation éventuelle du groupe hydroxy du C-22 dudit produit d'addition,
la réduction dudit produit d'addition pour obtenir un produit intermédiaire de formule: 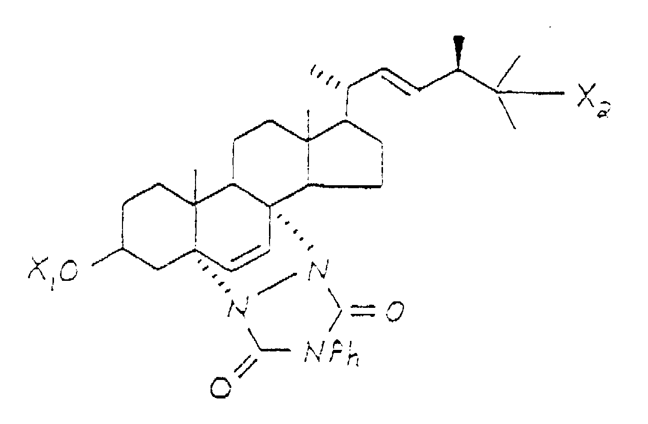 dans laquelle X₁ représente un atome d'hydrogène ou un groupe protégeant le groupe hydroxy, et X₂ représente un groupe hydroxy protégé,
et la transformation dudit produit intermédiaire de manière à obtenir la 25-hydroxy-24-épi-vitamine D₂, ladite étape de transformation comprenant l'élimination du groupe triazoline de façon à former un produit intermédiaire stéranique 5,7-diénique, la soumission dudit produit intermédiaire, en solution dans un éther ou un hydrocarbure, à l'action des rayons ultraviolets, l'isomérisation du dérivé de prévitamine D résultant, dans un solvant, à une température de 50°C à 90°C, et l'élimination ou l'échange puis l'élimination de tout groupe protecteur de groupe hydroxy.

9. Procédé selon la revendication 8, dans lequel on soumet en outre la 25-hydroxy-24-épi-vitamine D₂ à un procédé d'hydroxylation en 1α, de façon à obtenir le dérivé de vitamine D 1α hydroxylé correspondant.

10. Procédé selon la revendication 9, dans lequel le dérivé est la 1α,25-dihydroxy-24-épi-vitamine D₂.
